Europäisches Patentamt

European Patent Office

Office européen des brevets

(11) Veröffentlichungsnummer: **0 085 147**
**B1**

(12) **EUROPÄISCHE PATENTSCHRIFT**

(45) Veröffentlichungstag der Patentschrift:
10.07.85

(21) Anmeldenummer: **82110500.4**

(22) Anmeldetag: **13.11.82**

(51) Int. Cl.⁴: **A 61 F 2/32**

(54) Gerader, blattartiger Schaft für eine Gelenkendoprothese.

<table>
<tr><td>

(30) Priorität: **29.01.82 CH 541/82**

(43) Veröffentlichungstag der Anmeldung:
**10.08.83 Patentblatt 83/32**

(45) Bekanntmachung des Hinweises auf die Patenterteilung:
**10.07.85 Patentblatt 85/28**

(84) Benannte Vertragsstaaten:
**AT BE DE FR GB IT NL**

(56) Entgegenhaltungen:
**EP - A - 0 025 814**
**EP - A - 0 027 159**
**FR - A - 1 278 359**
**FR - A - 2 295 730**
**GB - A - 2 028 137**

</td><td>

(73) Patentinhaber: **GEBRÜDER SULZER AKTIENGESELLSCHAFT, Zürcherstrasse 9, CH-8401 Winterthur (CH)**
Patentinhaber: **Protek AG, Stadtbachstrasse 64, CH-3001 Bern (CH)**

(72) Erfinder: **Müller, Maurice E., Prof. Dr.-med., Inselspital, CH-3000 Bern (CH)**
Erfinder: **Niederer, Peter Gino, dipl.-Ing. ETH, Reichenbachstrasse 6, CH-3052 Zollikofen (CH)**
Erfinder: **Frey, Otto, Walrütlstrasse 56, CH-8400 Winterthur (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

</td></tr>
</table>

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft einen geraden, blattartigen Schaft für eine Gelenkendoprothese, insbesondere eine Hüftgelenkprothese, dessen Blattseiten sich vom distalen freien Ende zunächst symmetrisch zu einer Längsmittelachse konisch erweitern, wobei der Konus entlang der lateralen Schmalseite auf etwa $3/4$ der Schafthöhe endet, während medial die Erweiterung der Blattseiten aus dem Konus heraus in einer stetig gekrümmten Kurve in den den Gelenkkopf tragenden Prothesenhals mündet.

Prothesenschäfte der genannten Art für die Verankerung von Knochenimplantaten, insbesondere von Hüftgelenkprothesen, sind beispielsweise bekannt aus der CH-A-622 423; sie dienen vor allem zur zementfreien oder zumindest zementarmen Verankerung einer Prothese, wobei bei einer zementarmen Verankerung der als Füllmaterial dienende Knochenzement von einer tragenden Funktion entlastet ist und auch bei dieser Verankerungsart die tragende Abstützung weitestgehend durch Verklemmen des Schaftes im Knochen erfolgt.

Weiterhin ist aus der FR-A-1 278 359 eine Hüftgelenksprothese bekannt, bei der ein leicht gebogener Schaft mit Hilfe eines Keiles, dessen laterale Begrenzung parallel zur lateralen Seite des eigentlichen Schaftes verläuft, lateral an einer Stelle der Kortikalis verkeilt wird. Der Keil ist dabei in einer Führung auf der lateralen Seite des Schaftes geführt, in die Spongiosa eingeschlagen und drückt das distale Ende des Schaftes gegen die Kortikalis auf der medialen Seite des Knochenhohlraumes. Der kappenförmige Kopf der Prothese stützt sich auf einem verbliebenen Teil des natürlichen Femurkopfes ab, um die Einhaltung eines funktionsgerechten Höhenniveaus des Prothesenkopfes zu gewährleisten.

Die mehr oder weniger punktförmige Fixierung an der Kortikalis ergibt keine dauerhafte Verankerung des Schaftes, so daß es sehr häufig zu Schaftlockerungen, besonders bei Knochenabbau infolge von »Spitzen«-Belastungen und Mikrobewegungen kommt.

Um eine funktionsgerechte Lage des Gelenkkopfes der Prothese sicherzustellen, muß die Prothese bekanntlich so implantiert werden, daß das Zentrum des Gelenkkopfes sich etwa auf der Höhe der Spitze des großen Trochanters befindet. Da, wie vorstehend erwähnt, der Schaft vor allem durch Verklemmen fixiert ist, bereitet die Einhaltung der vorstehenden Forderung dem Operateur sehr oft Schwierigkeiten.

Aufgabe der Erfindung ist es daher, die Fixierung des Gelenkkopfes in einer bestimmten Höhe zu erleichtern; diese Aufgabe wird nach der Erfindung dadurch gelöst, daß der Schaft aus dem eigentlichen Blatt und einem sich über die ganze Schaftlänge erstreckenden keilförmigen, lateralen Endstück besteht, das, in einer Führung der lateralen Schmalseite des Blattes gleitend, auf das Blatt aufschiebbar ist.

Bei der neuen Prothese kann das den Gelenkkopf tragende Blatt zuerst in den vorbereiteten Knochen eingeschlagen und in einer Lage festgehalten werden, in der der Gelenkkopf die vorgeschriebene Höhe gegenüber dem großen Trochanter hat. Anschließend kann das Endstück auf die Führung des Blattes aufgeschoben und bei festgehaltenem Blatt eingeschlagen werden, wobei die Keilform des Endstückes ein Verkeilen des ganzen Schaftes im Knochen bewirkt, ohne daß der Gelenkkopf bzw. das Schaftblatt in ihrer Höhenlage verändert werden.

Ein weiterer Vorteil der neuen Konstruktion liegt darin, daß eine Serie verschiedener Schaftbreiten mit ein oder zwei Schaftblättern und einer Anzahl verschieden breiter Endstücke erheblich wirtschaftlicher bereitgestellt werden kann, als bisher, wo für jede Schaftbreite eines Satzes ein eigener Schaft gefertigt werden mußte; weiterhin sind mit der neuen Prothese unter Umständen kleinere Korrekturen, beispielsweise bei Schaftlockerungen, auch zu einem späteren Zeitpunkt nach der Implantation ohne großen Aufwand möglich, indem in einem kleinen Eingriff das Endstück — ebenfalls wiederum bei festgehaltenen Blatt — nochmals eingeschlagen wird.

Das Einschlagen des keilförmigen Endstückes wird erleichtert, wenn die Führung parallel zur Längsmittelachse des Schaftes verläuft. Um die Fixierung des Endstückes im Knochen zu verbessern, ist es zweckmäßig, wenn das Endstück längs seiner konischen Erweiterung mit wiederhakenartigen Verzahnungen versehen ist, wobei diese Verzahnungen vorteilhafterweise in den beiden Ecken des Übergangs von einer Breitseite zur lateralen Schmalseite angeordnet sind.

Um am proximalen Ende des Schaftes ein Einwachsen des Gewebes von oben in die Führung zu erschweren, ist es weiterhin sinnvoll, wenn die laterale Schmalseite der Endstücke im oberen proximalen Bereich der Schafthöhe parallel zur Längsmittelachse verläuft; diese Maßnahme hat zusätzlich den Vorteil, daß die Übertragung der Rotationskräfte vom Trochanter auf das Implantat und umgekehrt verbessert wird.

Um bei nicht völlig auszuschließenden Reoperationen das Ausziehen des Endstückes zu erleichtern, kann dieses an seinem oberen Ende für den Eingriff eines Auszieh-Instrumentes vorbereitet sein.

Als Führung eignen sich ganz allgemein einander übergreifende Elemente, die ein seitliches Abgleiten des Endstückes von der lateralen Schmalseite des Blattes verhindern, wobei es zweckmäßig ist, die gegenseitige Haftung von Blatt und Endstück durch eine möglichst große Reiboberfläche sicherzustellen; die Führung weist darüber hinaus — wie in der Implantat-Technik üblich — abgerundete Formen auf, um Belastungsspitzen im Implantat oder Knochen zu vermeiden.

Als Materialien für das Blatt und das Endstück dienen in erster Linie die in der Implantat-Technik gebräuchlichen Metalle und Metall-Legierungen; jedoch ist es auch möglich, beide Teile aus keramischem Material oder Kunststoff herzustellen, die gegebenenfalls mit geeigneten Überzügen versehen werden. Um ein kaltes Verschweißen der Führungsflächen von Blatt und Endstück während des Einschlagens des Endstückes zu vermeiden, ist es vorteilhaft, beide aus unterschiedlichen Metallen, die nicht zum Kaltschweißen neigen, wie z. B. Titan und einer Co-Basislegierung, zu fertigen. Es ist jedoch zu diesem Zweck auch möglich, eine oder beide Führungsflächen mit einer geeigneten Hartschicht, z. B. aus TiN, zu versehen.

Im folgenden wird die Erfindung anhand eines Ausführungsbeispiels im Zusammenhang mit der Zeichnung näher erläutert.

Fig. 1 zeigt in einem Längsschnitt durch das obere Ende eines Femurknochens eine in diesem fixierte Hüftgelenkprothese mit dem erfindungsgemäßen Schaft.

Fig. 2 ist der Schnitt II-II von Fig. 1.

Fig. 3 gibt einen Bereich A aus Fig. 1 in größerem Maßstab wieder.

Fig. 4 stellt in gleicher Ansicht wie Fig. 1 eine Ausführungsform des keilförmigen Endstückes dar, während in

Fig. 5a und 5b weitere Möglichkeiten für das Eingreifen eines Auszieh-Instrumentes in das obere Ende des Endstückes gezeigt sind.

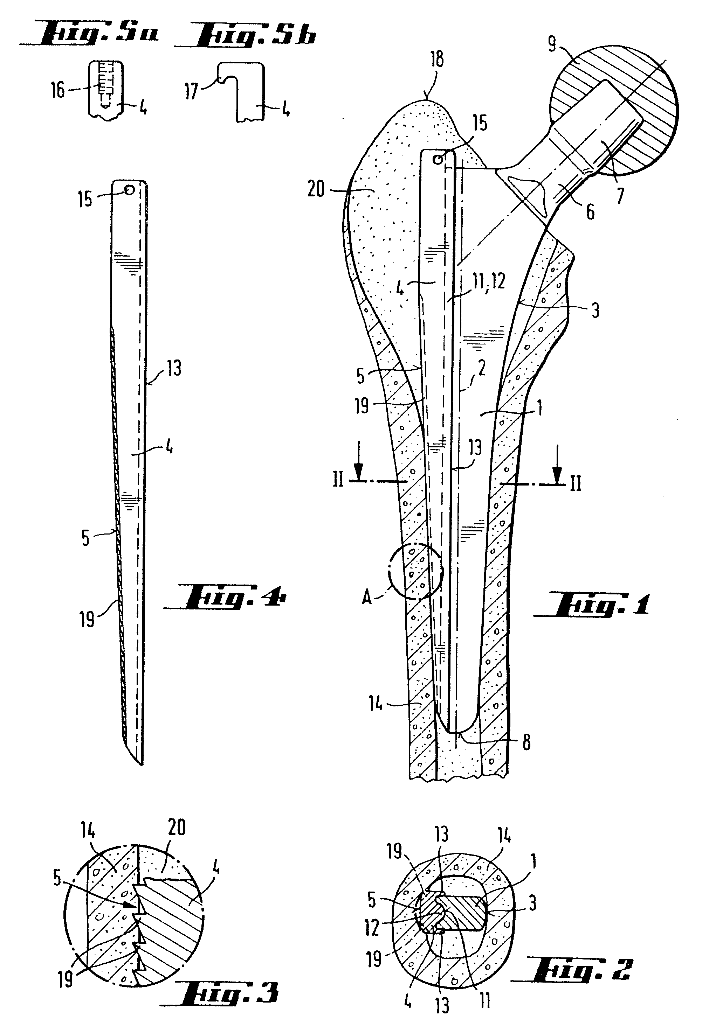

Der blattartige Prothesenschaft (Fig. 1), der sich von seinem distalen Ende 8 nach allen Seiten konisch erweitert, setzt sich gemäß der Erfindung zusammen aus einem eigentlichen Blatt 1 und einem an dessen laterale Schmalseite angesetzten, keilförmig verlaufenden Endstück 4; sein Konus ist symmetrisch zu einer Längsmittelachse 2 ausgebildet. Die mediale Schmalseite 3 des Konus geht in einen Bogen über, der in einem Hals 6 endet. Auf diesen ist ein sich nach außen konisch verjüngender Zapfen 7 aufgesetzt, der einen kugelförmigen Gelenkkopf 9 aufnimmt. Die Achse des Zapfens 7 schneidet die Längsmittelachse 2 des Schaftes unter einem Winkel, der im wesentlichen dem Winkel zwischen dem Schenkelhals der Femurachse eines natürlichen Hüftgelenkes entspricht und im vorliegenden Fall 45° beträgt.

Das Blatt 1 und das Endstück 4 sind über eine Führung miteinander verbunden; diese besteht aus einer allseitig abgerundeten parallel zur Längsachse 2 verlaufenden Hohlkehle 11 in der lateralen Schmalseite des Blattes 1. Als Gegenstück zu dieser Hohlkehle 11 hat das Endstück 4 in der Mitte eine vorspringende kufenartige Längsleiste 12. Weiterhin umfaßt es mit seinen Rändern 13 die Seitenflanken der Hohlkehle 11. Auf diese Weise wird das Endstück 4 beim Einschlagen so geführt, daß ein seitliches Abgleiten nicht möglich ist.

Wie bereits erwähnt, werden — um Kaltverschweißen zu verhindern — das Schaftblatt 1 und das Endstück 4 aus verschiedenen Materialien hergestellt, wobei das Blatt 1 beispielsweise aus einer Co-Basislegierung besteht, während das Endstück 4 aus Titan gefertigt ist; es kann jedoch auch mit einer Hartschicht auf Titan-Basis zumindest entlang der Führung belegt sein.

Auf der lateralen Schmalseite 5 des zusammengesetzten Schaftes bzw. des Endstückes 4 reicht die Erweiterung des Konus bis auf etwa $2/3$ bis $3/4$ der Schafthöhe; von dort aus verläuft die laterale Schmalseite 5 des Endstückes 4 parallel zur Längsmittelachse 2.

Die beiden Übergänge von der »Breitseite« des Endstückes 4 zur lateralen Schmalseite 5 sind über die Höhe des Konus mit je einer Verzahnung 19 versehen, die nach dem Einschlagen des Endstückes 4 widerhakenartig in die kortikale Knochensubstanz 14 eingreift und so ein unbeabsichtigtes Herausziehen des keilförmigen Endstückes 4 verhindert.

Für den Eingriff eines Ausziehinstrumentes hat das Endstück 4 an seinem oberen oder proximalen Ende eine Öse 15; diese kann jedoch durch andere geeignete Verbindungselemente, wie z. B. ein Gewinde 16 (Fig. 5a) oder einen hakenartigen Ansatz 17 (Fig. 5b) ersetzt sein.

Wie bereits erwähnt, erlaubt die neue Schaftkonstruktion zunächst das den Gelenkkopf 9 tragende Blatt 1 in das operativ vorbereitete, spongiöse Knochengewebe 20 soweit einzuschlagen, daß das Zentrum des Gelenkkopfes 9 etwa auf der Höhe der Trochanterspitze 18 liegt, und dann das Blatt 1 bzw. der Gelenkkopf 9 festzuhalten und die Prothese durch Einschlagen des Endstückes 4 zu fixieren, wobei das Blatt 1 und das Endstück 4 miteinander zwischen einander gegenüberliegenden Bereichen des kortikalen Gewebes 14 gegeneinander verkeilt werden.

## Patentansprüche

1. Gerader, blattartiger Schaft für eine Gelenkendoprothese, insbesondere eine Hüftgelenkprothese, dessen Blattseiten sich vom distalen freien Ende (8) zunächst symmetrisch zu einer Längsmittelachse (2) konisch erweitern, wobei der Konus entlang der lateralen Schmalseite (5) auf etwa $3/4$ der Schafthöhe endet, während medial die Erweiterung der Blattseiten aus dem Konus heraus in einer stetig gekrümmten Kurve in den den Gelenkkopf (9) tragenden Prothesenhals (6) mündet, dadurch gekennzeichnet, daß der Schaft aus dem eigentlichen Blatt (1) und einem sich über die ganze Schaftlänge erstreckenden keilförmigen, lateralen Endstück (4) besteht, das in einer Führung (11, 12, 13) der lateralen Schmalseite (5) des Blattes (1) gleitend, auf das Blatt (1) aufschiebbar ist.

2. Schaft nach Anspruch 1, dadurch gekennzeichnet, daß die Führung (11, 12, 13) parallel zur Längsmittelachse (2) des Schaftes verläuft.

3. Schaft nach Anspruch 1 oder 2, dadurch gekennzeichnet, daß das Endstück (4) längs seiner konischen Erweiterung mit widerhakenartigen Verzahnungen (19) versehen ist.

4. Schaft nach Anspruch 3, dadurch gekennzeichnet, daß die Verzahnungen (19) in den beiden Ecken des Übergangs von einer Breitseite zur lateralen Schmalseite (5) angeordnet ist.

5. Schaft nach Anspruch 1, dadurch gekennzeichnet, daß die laterale Schmalseite (5) des Endstückes (4) im oberen proximalen Bereich der Schafthöhe parallel zur Längsmittelachse (2) verläuft.

6. Schaft nach einem der vorhergehenden Ansprüche, dadurch gekennzeichnet, daß das Endstück (4) an seinem oberen Ende für den Eingriff eines Ausziehinstrumentes vorbereitet ist.

**Claims**

1. A straight blade-like intramedullary stem for a joint endoprosthesis, more particularly a hip joint prothesis, the sides of the stem first widening conically from the distal free end (8) symmetrically of a longitudinal central axis (2), the cone ending along the lateral narrow side (5) at about threequarters of stem height while on the medial side the widening of the blade sides extends from the cone in a continuous curve into the prosthesis neck (6) carrying the joint head (9), characterised in that the stem comprises the blade (1) and a wedge-shaped lateral end member (4) which extends over the whole length of the stem and which, sliding in a guide (11, 12, 13) of the lateral narrow side (5) of the blade (1), can be pushed on to the blade (1).

2. A stem according to claim 1, characterised in that the guide (11, 12, 13) extends parallel to the longitudinal central axis (2) of the stem.

3. A stem according to claim 1 or 2, characterised in that the end member (4) has along its conical widened part barb-like toothings (19).

4. A stem according to claim 3, characterised in that the toothings (19) are disposed in the two corners of the transition between a wide side and the lateral narrow side (5).

5. A stem according to claim 1, characterised in that the lateral narrow side (5) of the end member (4) extends parallel to the longitudinal central axis (2) in the top proximal zone of stem height.

6. A stem according to any of the previous claims, characterised in that the end member (4) is prepared at its top end for the engagement of a withdrawing instrument.

**Revendications**

1. Broche rectiligne du type spatule pour une endoprothèse articulée, en particulier une prothèse coxofémorale, dont les faces de la spatule s'évasent tout d'abord tronconiquement à partir de l'extrémité libre distale (8), symétriquement par rapport à un axe médian longitudinal (2), le cône longeant la face latérale étroite (5) s'achevant environ aux 3/4 de la hauteur de la broche, tandis que, du côté médial, l'évasement des faces de la spatule au-delà du cône débouche, selon un arc de courbure permanente, dans le col (6) de la prothèse supportant la tête d'articulation (9), caractérisée par le fait que la broche se compose de la spatule (1) proprement dite ainsi qui d'une pièce latérale cunéiforme d'extrémité (4) qui, s'étendant sur toute la longueur de la broche, peut être enfilée sur la spatule (1) en coulissant dans un guidage (11, 12, 13) de la face latérale étroite (5) de cette spatule (1).

2. Broche selon la revendication 1, caractérisée par le fait que le guidage (11, 12, 13) s'étend parallèlement à l'axe médian longitudinal (2) de cette broche.

3. Broche selon la revendication 1 ou 2, caractérisée par le fait que la pièce d'extrémité (4) est dotée, le long de son évasement tronconique, de dentures (19) du type ardillons.

4. Broche selon la revendication 3, caractérisée par le fait que les dentures (19) sont disposées dans les deux coins de la transistion entre une face large et la face latérale étroite (5).

5. Broche selon la revendication 1, caractérisée par le fait que la face latérale étroite (5) de la pièce d'extrémité (4) s'étend, dans la région proximale supérieure de la hauteur de la broche, parallèlement à l'axe médian longitudinal (2).

6. Broche selon l'une quelconque des revendications précédentes, caractérisée par le fait que la pièce d'extrémité (4) est apprêtée, à son extrémité supérieure, pour la venue en prise d'un instrument d'extraction.

**Fig. 5a**

16 — 4

**Fig. 5b**

17 — 4

15

13

4

5

19

**Fig. 4**

18

9

15

20

4

11,12

3

5

2

19

1

13

II — II

13

A

14

8

**Fig. 1**

14

20

5

4

19

**Fig. 3**

19

13

14

5

1

12

3

19

4

13

11

**Fig. 2**